# EUROPEAN PATENT APPLICATION

(11) **EP 4 759 226 A1**
(43) Date of publication of application: **17.06.2026**
(21) Application number: 24897969.2
(22) Date of filing: 15.11.2024
(51) Int. Cl.: A61B 5/024, A61B 5/0245, A61B 5/346, A61B 5/28, A61B 5/256, A61B 5/00

(54) **ELECTRONIC DEVICE AND METHOD FOR GENERATING HEART RATE-RELATED DATA USING SAME**

(30) Priority: 29.11.2023 KR 20230169455; 03.01.2024 KR 20240001144
(71) Applicant: Samsung Electronics Co., Ltd., Suwon-si, Gyeonggi-do 16677 (KR)
(72) Inventor: KIM, Youngmi, Suwon-si, Gyeonggi-do 16677 (KR); BAE, Sanghee, Suwon-si, Gyeonggi-do 16677 (KR); JEON, Eunbee, Suwon-si, Gyeonggi-do 16677 (KR); KIM, Yookyung, Suwon-si, Gyeonggi-do 16677 (KR); LIM, Cheolkyu, Suwon-si, Gyeonggi-do 16677 (KR)
(74) Representative: HGF
(86) International application number: PCT/KR2024/018041
(87) International publication number: WO 2025/116373

(57) **Abstract**

An electronic device includes: a heart rate measurement sensor; at least one processor; and at least one memory including computer program code, where the at least one memory and the computer program code are configured, with the at least one processor, to cause the electronic device to at least: obtain heart rate information by using the heart rate measurement sensor, identify the obtained heart rate information and electrocardiogram (ECG)-related symptoms information corresponding to the heart rate information, and generate first data, based on the heart rate information and the ECG-related symptoms information.

## Description

### [Technical Field]

The disclosure relates to an electronic device and a method of generating data related to heart rate using the electronic device.

### [Background Art]

With the recent advancement of technology, electronic devices have gradually evolved from a uniform rectangular shape to a variety of shapes. For example, the electronic devices are transformed into wearable devices capable of being worn on a part of the body (e.g., hand, wrist, finger, head, neck, or ear) to improve portability or accessibility of the user. For example, wearable devices may include a watch-type device that is worn on the user's wrist and provide various user experiences and useful functions.

The above information may be provided as related art for the purpose of helping to understand the disclosure. No claim or determination is made as to whether any description above can be applied as prior art related to the disclosure.

### [Disclosure of Invention]

### [Technical Problem]

The electronic device may include or correspond to a wearable device at least partially mounted on a user's body, for example, a watch worn on a wrist. The wearable device may be disposed such that a biometric sensor for obtaining biometric information (e.g., heart rate information) of the user is at least partially in contact with a part of the user's body. The wearable device may obtain biometric information of the user using biometric sensors and measure a user's heart rate, based on the obtained biometric information. The electronic device may perform a heart rate measurement function to measure the user's heart rate and provide the user with information related to the measured heart rate.

It may be quite important to accurately measure the user's heart rate periodically or aperiodically for managing the user's health. In particular, accurate measurement and recording of the heart rate is essential in detecting or predicting arrhythmia, which is one of the diseases related to the heart rate. For example, arrhythmia may be caused when there is an abnormality in the regular movement of the heart that contracts and expands. Arrhythmia may include a situation where the heartbeat becomes abnormally fast, slow, or irregular. For example, when the arrhythmia occurs, the user may feel a pounding heart, dizziness, or increased fatigue.

An electronic device is configured to measure a user's heart rate using a heart rate measurement sensor and to identify the measured heart rate information and/or electrocardiogram (ECG)-related symptoms information (e.g., arrhythmia-related symptoms information) produced based on the heart rate information, and transmit the checked ECG-related symptoms information to a doctor.

The technical problem to be solved in this document is not limited to the technical problem mentioned above, and other technical problems not mentioned may be clearly understood by those skilled in the art to which the disclosure pertains from the description below.

### [Solution to Problem]

According to an aspect of the disclosure, an electronic device includes: a heart rate measurement sensor; at least one processor; and at least one memory including computer program code, where the at least one memory and the computer program code are configured, with the at least one processor, to cause the electronic device to at least: obtain heart rate information by using the heart rate measurement sensor, identify the obtained heart rate information and electrocardiogram (ECG)-related symptoms information corresponding to the heart rate information, and generate first data, based on the heart rate information and the ECG-related symptoms information.

According to an aspect of the disclosure, a method for generating data related to electrocardiogram, includes: obtaining heart rate information using a heart rate measurement sensor; identifying the obtained heart rate information and ECG-related symptoms information corresponding to the heart rate information; and generating first data, based on the heart rate information and the ECG-related symptoms information.

According to an aspect of the disclosure, a non-transitory computer-readable storage medium storing one or more programs for performing a method for generating an ECG-related file, wherein the one or more programs comprise instructions configured to, when executed by at least one processor of an electronic device, perform: obtaining heart rate information using a heart rate measurement sensor; identifying the obtained heart rate information and ECG-related symptoms information corresponding to the heart rate information; and generating first data, based on the heart rate information and the ECG-related symptoms information.

### [Advantageous Effects of Invention]

According to an embodiment, the electronic device may identify ECG-related symptoms (e.g., arrhythmia symptoms), based on measured heart rate information, when performing a heart rate measurement function using a heart rate measurement sensor. The electronic device may generate ECG-related data to be provided to a doctor (e.g., a medical professional), based on the identified ECG-related symptoms. For example, the electronic device may periodically or aperiodically measure a user's heart rate, obtain an external audio signal through an audio recording function when measuring the heart rate, and determine whether or not ECG-related symptoms occur, based on the measured heart rate and/or the obtained audio signal. The electronic device may generate data related to the electrocardiogram in which ECG-related symptoms occur when performing the heart rate measurement function.

According to an embodiment, the electronic device may detect ECG-related symptoms that abnormally occur, and generate ECG-related data, based on the detected ECG-related symptoms. The ECG-related data may be implemented in various file formats. For example, the user may detect abnormally occurring ECG-related symptoms and generate ECG-related data, based on the detected ECG-related symptoms. The user may share the generated ECG-related data with a doctor during subsequent treatment and manage ECG-related symptoms more conveniently. The user's convenience in measuring and managing the heart rate may be improved.

The effects obtainable from the disclosure are not limited to the effects mentioned above. Other effects that are not mentioned may be understood by those skilled in the art to which the disclosure belongs from the description below.

### [Brief Description of Drawings]

The above and other aspects, features, and advantages of certain embodiments of the disclosure will be more apparent from the following description taken in conjunction with the accompanying drawings, in which:
FIG. 1 is a block diagram of an electronic device in a network environment according to an embodiment of the disclosure;
FIG. 2A is a front perspective view of an electronic device according to an embodiment of the disclosure;
FIG. 2B is a rear perspective view of an electronic device according to an embodiment of the disclosure;
FIG. 3A illustrates an embodiment in which data related to a heart rate is generated based on heart rate information measured by a first electronic device and in which the data related to the heart rate is shared with a second electronic device according to an embodiment of the disclosure;
FIG. 3B is a block diagram of an electronic device according to an embodiment of the disclosure;
FIG. 4 illustrates a flowchart of a method of generating a first ECG file for identifying ECG-related symptoms, based on measured heart rate information, according to an embodiment of the disclosure;
FIG. 5 illustrates a flowchart of a method of generating a second ECG file for identifying ECG-related symptoms, based on measured heart rate information and an external audio signal, according to an embodiment of the disclosure;
FIG. 6 illustrates an example diagram of operations in respective steps in the process of generating an ECG file according to an embodiment of the disclosure;
FIG. 7 illustrates an example diagram of an operation of outputting configured questions in relation to ECG symptoms according to an embodiment of the disclosure;
FIG. 8 illustrates an example diagram of an operation of obtaining an audio signal of a user for a question related to ECG symptoms and recording ECG symptoms, based on the obtained user's audio signal, according to an embodiment of the disclosure; and
FIG. 9 illustrates an example diagram of an operation of identifying a predetermined telephone number according to an audio signal of a user and requesting a call to the predetermined telephone number according to an embodiment of the disclosure.

### [Mode for the Invention]

Hereinafter, embodiments of the disclosure will be described in detail with reference to the accompanying drawings such that those skilled in the art to which the disclosure pertains can easily practice the same. However, the disclosure may be implemented in various different forms without being limited to the embodiments described herein. In relation to the description of the drawings, identical or similar components may be given identical or similar reference numerals. In addition, in the drawings and related descriptions, descriptions of known functions and configurations may be omitted for clarity and brevity of description.

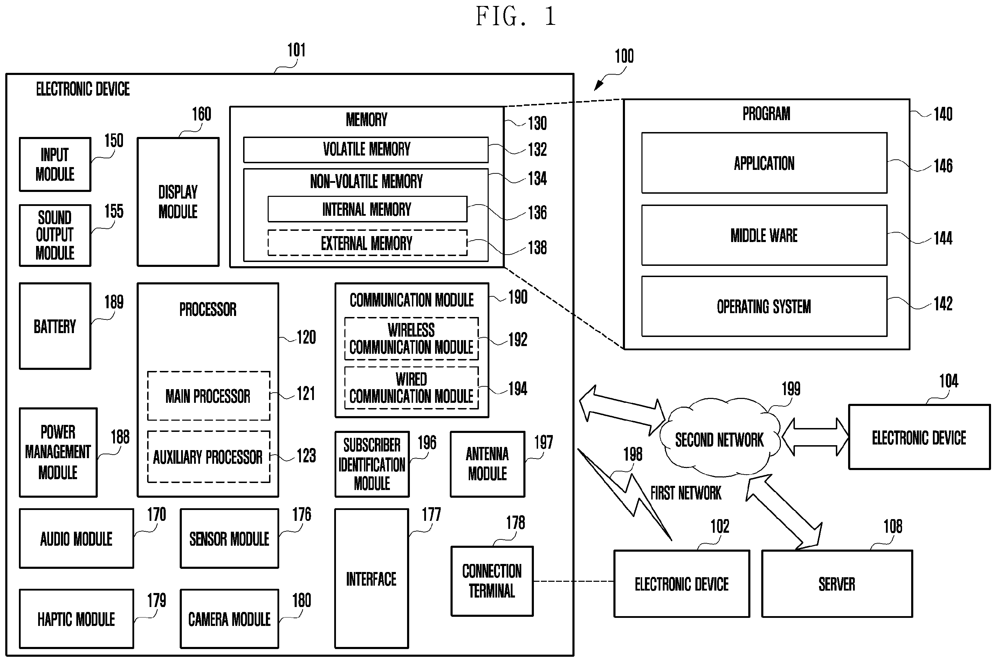

Fig. 1 is a block diagram illustrating an example electronic device 101 in a network environment 100 according to various embodiments. Referring to Fig. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to an embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semisupervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to an embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as BluetoothTM, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element including a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form an mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band. For example, the plurality of antennas may include a patch array antenna and/or a dipole array antenna.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra-low-latency services using, e.g., distributed computing or mobile edge computing. In an embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

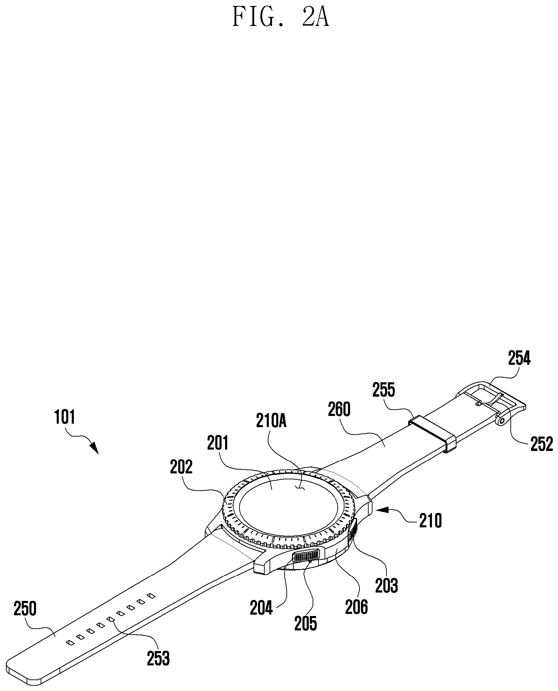

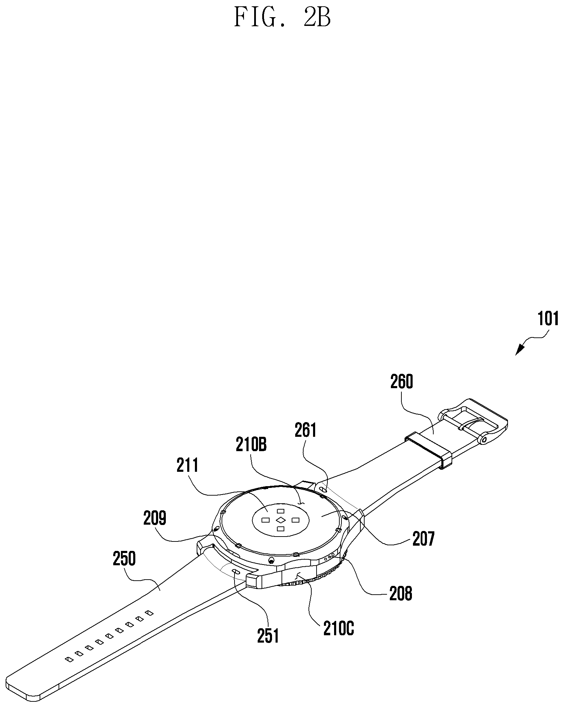

FIG. 2a is a front perspective view of an electronic device according to an embodiment of the disclosure. FIG. 2b is a rear perspective view of an electronic device according to an embodiment of the disclosure.

The electronic device 101 in FIG. 2a and FIG. 2b (for example, the electronic device 101 in FIG. 1) may be at least partially similar to the electronic device 101 in FIG. 1, or may further include other embodiments of the electronic device. For example, the electronic device 101 may include a wearable device or a wearable watch which can be worn on a part of a human body.

Referring to FIG. 2a and FIG. 2b, the electronic device 101 according to an embodiment (for example, a wearable device or a wearable watch) may include a housing 210 including a first surface (or front surface) 210A, a second surface (or rear surface) 210B, and a lateral surface 210C surrounding the space between the first surface 210A and the second surface 210B, and clamping members 250 and 260 connected to at least a part of the housing 210 and configured such that the electronic device 101 can be detachably clamped to a part of the user's body (for example, wrist or ankle). The clamping members 250 and 260 may be straps, for example, wound around the user's wrist such that the electronic device 101 is retained. In another embodiment, the housing may refer to a structure forming at least some of the first surface 210A, the second surface 210B, and the lateral sur5face 210C of the electronic device 101. According to an embodiment, at least a part of the first surface 210A may be formed by a substantially transparent front plate 201 (for example, a glass plate including various coating layers, or a polymer plate). The second surface 210B may be formed by a rear plate 207. The rear plate 207 may be formed by, for example, coated or colored glass, ceramic, polymer, metal (for example, aluminum, stainless steel (STS), or magnesium), or a combination of at least two of the above materials. The lateral surface 210C may be coupled to the front plate 201 and the rear plate 207, and may be formed by a lateral bezel structure (or (lateral member") 206 including a metal and/or a polymer. In some embodiments, the rear plate 207 and the lateral bezel structure 206 may be formed integrally, and may include the same material (for example, a metal material such as aluminum). The clamping members 250 and 260 may be made of various materials in various shapes. For example, integrated and multiple unit links may be formed to be movable with regard to each other by using, for example, a woven material, leather, rubber, synthetic resin, metal, ceramic, or a combination of at least two of the above materials.

According to an embodiment, the electronic device 101 may include at least one of a display (for example, the display module 160 in FIG. 1), audio modules 205 and 208 (for example, the audio module 170 in FIG. 1), a sensor module 211 (for example, the sensor module 176 in FIG. 1), key input devices 202, 203, and 204 (for example, the input module 150 in FIG. 1), and a connector hole 209. In some embodiments, at least one of the components (for example, the key input devices 202, 203, and 204, the connector hole 209, or the sensor module 211) of the electronic device 101 may be omitted, or the electronic device 101 may additionally include other components. According to an embodiment, the components are not limited to the parts illustrated in FIG. 2a and FIG. 2b.

The display module 160 may be visually exposed, for example, through a corresponding portion of the front plate 201. For example, the user may identify at least one content displayed on the display module 160 through the front plate 201. The display module 160 may have a shape corresponding to the shape of the front plate 201, which may be one of a circle, an ellipse, and/or a polygon. The display module 160 may be at least partially coupled to or disposed adjacent to a touch sensing circuit, a pressure sensor capable of measuring the intensity (pressure) of a touch, and or a fingerprint sensor.

The audio modules 205 and 208 may include a microphone hole 205 and a speaker hole 208. The microphone hole 205 may have a microphone disposed therein so as to acquire external sounds. In some embodiments, multiple microphones may be disposed such that the direction of sounds can be sensed. The speaker hole 208 may be used as an external speaker and a telephone speech receiver. In some embodiments, the speaker hole 208 and the microphone hole 205 may be implemented as a single hole, or a speaker (for example, a piezoelectric speaker) may be included without the speaker hole 208.

The sensor module 211 may generate an electric signal or a data value corresponding to the internal operating state of the electronic device 101, or external environment states. The sensor module 211 may include a biometric sensor module (for example, a biometric sensor, a heart rate monitor (HRM) sensor, an oxygen saturation sensor, and/or a blood glucose sensor) disposed toward the second surface 210B of the housing 210, for example. When the electronic device 101 is worn on a part of a human body (for example, on a wrist), the sensor module 211 may be disposed so as to at least partially contact the human body. For example, if the electronic device 101 is worn on the wrist, the sensor module 211 may be disposed so as to be in at least partially physical contact with the skin of the wrist, and may obtain biometric information (e.g., heart rate information) of the user through the skin. The electronic device 101 may further include a sensor module, for example, at least one of a gesture sensor, a gyro sensor, a barometric pressure sensor, a magnetic sensor, an acceleration sensor, a grip sensor, a color sensor, an infrared (IR) sensor, a biometric sensor, a temperature sensor, a humidity sensor, or an illuminance sensor.

The key input devices 202, 203, and 204 may include a wheel key 202 disposed so as to correspond to the first surface 210A of the housing 210 and to be able to rotate along at least one direction (for example, clockwise, counterclockwise), and/or side key buttons 203 and 204 disposed on the lateral surface 210C of the housing 210. The wheel key 202 may have a shape corresponding to the shape of the front plate 201. According to another embodiment, the electronic device 101 may not include some or all of the above-mentioned key input devices 202, 203, and 204, and the key input devices 202, 203, and 204 not included may be implemented on the display 220 as soft keys or touch keys.

The connector hole 209 may contain a connector (for example, a USB connector) for transmitting/receiving power and/or data with an external electronic device (for example, the electronic devices 102 and 104 in FIG. 1). The electronic device 101 may further include, for example, a connector cover configured to cover at least a part of the connector hole 209 and to prevent external foreign materials from infiltrating the connector hole 209.

The clamping members 250 and 260 may be detachably clamped in at least a partial region of the housing 210 by using locking members 251 and 261. The clamping members 250 and 260 may include at least one of a retaining member 252, a retaining member fastening hole 253, a band guide member 254, and a band retaining ring 255. According to an embodiment, the electronic device 101 may remain clamped at least partially to a part of a human body (for example, on a wrist) by using the clamping members 250 and 260.

The retaining member 252 may be at least partially coupled to the retaining member fastening hole 253 such that the housing 210 and the clamping members 250 and 260 are retained on a part of the user's body (for example, on a wrist or an ankle). The band guide member 254 may be configured such that, when the retaining member 252 is fastened to the retaining member fastening hole 253, the range of movement of the retaining member 252 is limited, thereby guaranteeing that the electronic device 101 is clamped to a part of the human body while the clamping members 250 and 260 maintain close contact with a part of the user's body. The band retaining ring 255 may limit the range of movement of the clamping members 250 and 260 while the retaining member 252 and the retaining member clamping hole 253 remain fastened to each other.

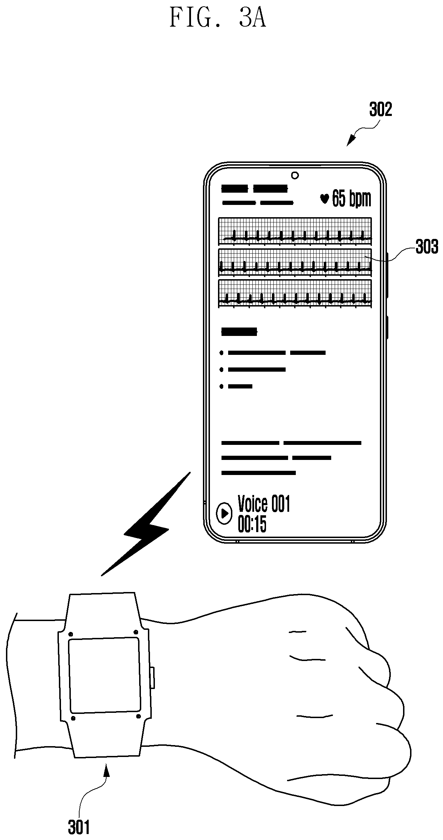

FIG. 3A illustrates a diagram about an embodiment in which data related to a heart rate is generated based on heart rate information measured by a first electronic device and in which the data related to the heart rate is shared with a second electronic device, according to an embodiment of the disclosure.

A first electronic device 301 (e.g., a wearable device or a watch) in FIG. 3A may be at least partially similar to the electronic device 101 in FIG. 1 or may further include other embodiments of the electronic device 101. A second electronic device 302 (e.g., a smartphone or a portable terminal) in FIG. 3A may be at least partially similar to the electronic device 102 or 104 in FIG. 1, or may further include other embodiments of the electronic device 102 or 104. The operations illustrated in FIG. 3A may be performed in at least one of the first electronic device 301 and/or the second electronic device 302. According to an embodiment, some (e.g., an operation of measuring heart rate information) of the operations illustrated in FIG. 3A may be performed in the first electronic device 301, and others (e.g., an operation of generating data related to a heart rate, based on the measured heart rate information) of the operations illustrated in FIG. 3A may be performed in the second electronic device 302. According to an embodiment, the operations illustrated in FIG. 3A may be performed independently in each of the first electronic device 301 and the second electronic device 302.

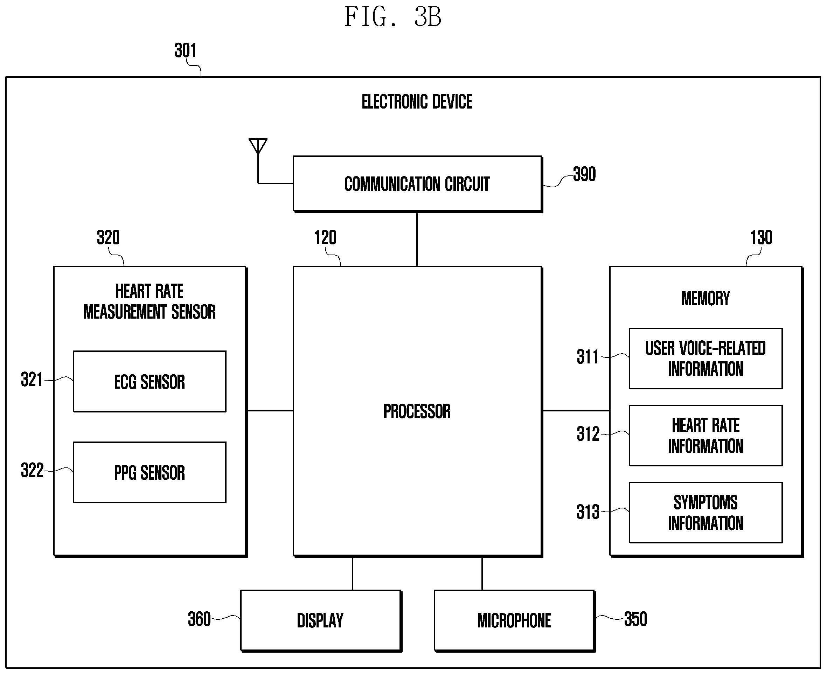

Referring to FIG. 3A, the first electronic device 301 may include a wearable device (e.g., a smart watch or a smart ring) capable of being worn on a user's body (e.g., a wrist, a finger), and may measure and obtain biometric information of the user (e.g., heart rate information or a user's heart rate) using a heart rate measurement sensor (e.g., the heart rate measurement sensor 320 in FIG. 3B). The wearable watch (the smart watch) illustrated in FIGS. 2A and 2B are not the only example of the first electronic device 301 described in the disclosure. Other form factors that can be worn by or attached to the user, such as the smart ring, can be other examples of the first electronic device 301. The first electronic device 301 may generate data (e.g., a file in a form capable of being shared with an external electronic device) related to the user's heart rate, based on the user's biometric information (e.g., heart rate information). For example, the data related to the heart rate may be implemented in various file forms. The first electronic device 301 may be operatively connected to the second electronic device 302 (e.g., portable terminal) through a communication circuit (e.g., the communication module 190 in FIG. 1), and may provide biometric information (e.g., user's heart rate information or a heart rate-related file in which the heart rate information is identified) measured by the first electronic device 301 to the second electronic device 302. The second electronic device 302 may display a user interface 303 related to ECG, based on the biometric information (e.g., heart rate information or information related to the heart rate) provided from the first electronic device 301.

According to an embodiment, the first electronic device 301 may include a wearable watch capable of being worn on a part of the user's body (e.g., wrist), and may include a heart rate measurement sensor (e.g., the sensor module 211 in FIG. 2B or the heart rate measurement sensor 320 in FIG. 3B) for measuring the user's heart rate through the skin in physical contacts with the same. For example, the heart rate measurement sensor 320 may be disposed in the first electronic device 301 so as to be at least partially exposed to the external environment. For example, the heart rate measurement sensor 320 may include an ECG sensor for detecting an electric signal depending on a heartbeat and/or a photoplethysmography (PPG) sensor for measuring blood flow by using light. The first electronic device 301, in response to (or based on) the execution of a heart rate measurement function, may at least partially activate the heart rate measurement sensor 320 and measure the heart rate of the user using the activated heart rate measurement sensor 320. According to an embodiment, the first electronic device 301 may perform the heart rate measurement function periodically or aperiodically and measure the heart rate of the user according to the execution of the heart rate measurement function. The first electronic device 301 may perform the heart rate measurement function to obtain heart rate information corresponding to the measured heart rate. According to an embodiment, the first electronic device 301 may generate data related to the heart rate, based on the obtained heart rate information, and share the data related to the heart rate with another electronic device. For example, the data related to the heart rate may be implemented in a variety of file formats.

According to an embodiment, the first electronic device 301 may be operatively connected to the second electronic device 302 (e.g., portable terminal) through a communication circuit (e.g., the communication circuit 390 in FIG. 3B), and may share the generated heart rate-related data with the second electronic device 302. According to an embodiment, the user of the first electronic device 301 may share the heart rate-related data (e.g., heart rate file) generated based on the user's heart rate information with others (e.g., doctors and other electronic devices), and the heart rate-related data (e.g., heart rate file) may be utilized as medical treatment information of the user. For example, when the shared heart rate-related data is executed in the second electronic device 302, the second electronic device 302 may display a user interface 303 corresponding to the heart rate-related data.

According to an embodiment, when executing the heart rate measurement function, the first electronic device 301 may at least partially activate a microphone (e.g., the microphone 350 in FIG. 3B) and execute an audio recording function by the microphone. For example, the first electronic device 301 may obtain an external audio signal using the microphone and identify ECG symptoms information (e.g., information related to ECG symptoms), based on the obtained audio signal. The audio signal may include a voice signal (e.g., natural language) and/or a non-voice signal (e.g., voice tremor symptom, moaning sound, breathing sound, change in tone, or sound related to a fall) from the user. The first electronic device 301 may determine whether or not symptoms related to ECG (e.g., symptoms information related to arrhythmia) occur, based on the obtained audio signal. For example, the first electronic device 301 may convert the obtained audio signal (e.g., voice signal) into text and, if the converted text contains a word related to arrhythmia symptoms, determine that the user has experienced arrhythmia-related symptoms. For another example, the first electronic device 301 may analyze the obtained audio signal (e.g., non-voice signal) and, based on the analyzed audio signal, determine whether or not a predetermined phenomenon occurs (e.g., occurrence of voice tremor, moans, breathing difficulty, and/or abnormal tone change).

According to an embodiment, the first electronic device 301 (e.g., wearable device) may automatically execute an audio recording function through the microphone 350 when performing a heart rate measurement function. The first electronic device 301 may determine whether or not a symptom related to arrhythmia occurs in the user, based on the heart rate information measured by using a heart rate measurement sensor (e.g., the heart rate measurement sensor 320 in FIG. 3B) and/or audio information obtained by the audio recording function. If the occurrence of a symptom related to arrhythmia is identified, the first electronic device 301 may generate heart rate-related data (e.g., heart rate file or electrocardiogram file) based on the arrhythmia symptom and share the generated heart rate-related data with the second electronic device 302 (e.g., portable terminal).

According to an embodiment, the first electronic device 301 may detect symptoms information related to arrhythmia that occurs periodically or aperiodically and generate heart rate-related data (e.g., ECG file), based on the symptoms information related to arrhythmia. For example, in a hospital treatment, a user of the first electronic device 301 may pass the generated ECG file to a doctor and hear an explanation of symptoms information related to arrhythmia from the doctor. According to an embodiment, the user may conveniently generate the heart rate-related data (e.g., ECG file) including symptoms information related to arrhythmia, and conveniently manage his or her health.

FIG. 3B is a block diagram of an electronic device according to an embodiment of the disclosure.

The electronic device 301 in FIG. 3B may be at least partially similar to the electronic device 101 in FIG. 1, or may further include other embodiments of the electronic device 101. The electronic device 301 in FIG. 3B may include a wearable device (e.g., watch) capable of being worn on a part of the human body (e.g., wrist) such as the first electronic device 301 in FIG. 3A.

Referring to FIG. 3B, the electronic device 301 may include a processor (e.g., the processor 120 in FIG. 1), a memory (e.g., the memory 130 in FIG. 1), a heart rate measurement sensor 320 (e.g., the sensor module 176 in FIG. 1), a microphone 350 (e.g., the input module 150 in FIG. 1), a display 360 (e.g., the display module 160 in FIG. 1), and/or a communication circuit 390 (e.g., the communication module 190 in FIG. 1).

According to an embodiment, the processor 120 of the electronic device 301 may execute a program (e.g., the program 140 in FIG. 1) stored in the memory 130 to control at least one other component (e.g., hardware or software component) and perform various data processing or operations. According to an embodiment, the processor 120 may be operatively, functionally, and/or electrically connected to the memory 130, the heart rate measurement sensor 320, the microphone 350, the display 360, and/or the communication circuit 390.

According to an embodiment, the memory 130 may store a program (e.g., an application program or a health management application) related to heart rate measurement for measuring and managing a heart rate of the user. For example, the processor 120 may measure the heart rate of the user, based on the program related to heart rate measurement and determine whether or not ECG symptoms (e.g., symptoms related to ECG) occur in the user, based on the measured heart rate. According to an embodiment, the processor 120 may perform health management according to the heart rate of the user, based on the program related to heart rate measurement.

According to an embodiment, the memory 130 may include user's voice-related information 311, heart rate information 312, and symptoms information 313. The user's voice-related information 311 may include information related to the mother tongue of the user, information related to the user's voice, and/or information related to the user's pronunciation. For example, the processor 120 may analyze an audio signal (e.g., natural language), based on the user's voice-related information 311, and convert the audio signal into text. According to an embodiment, even if the user arbitrarily utters sentences and/or words, the processor 120 may convert the uttered audio signal into text, based on the user's voice-related information 311. The heart rate information 312 may include information related to the user's heart rate (e.g., heart rate per minute, average heart rate, and heart rate in specific situations). The heart rate information 312 may include various information related to the user's heart rate. For example, the processor 120 may predict and guess the current status of the user, based on the heart rate information 312, and determine whether or not the user has experienced symptoms related to arrhythmia. The symptoms information 313 may include at least one piece of symptoms information related to arrhythmia. For example, the symptoms related to arrhythmia may include at least one of a symptom in which a measured heart rate exceeds a predetermined heart rate, a symptom of an irregular heartbeat, a symptom in which the heartbeat interval is irregular, and/or a symptom causing suspicion of diseases related to arrhythmia. The symptoms related to arrhythmia may be stored as text in the form of a list.

According to an embodiment, the heart rate measurement sensor 320 may include an ECG sensor 321 (e.g., ECG sensor) for detecting an electrical signal depending on a heartbeat and/or a PPG sensor 322 (e.g., an optical blood flow measurement sensor, an optical volumetric pulse measurement sensor, or an optical volumetric measurement sensor) for measuring blood flow by using light. For example, the ECG sensor 321 may detect an electrical signal according to a heartbeat while at least partially in contact with the skin. The electronic device 301 may implement a graph related to the user's heart rate (e.g., a graph representing electrical activity of the heart) using the ECG sensor 321. According to an embodiment, the processor 120 may detect an electrical signal depending on a heartbeat, based on the ECG sensor 321 included in the heart rate measurement sensor 320, and measure a heart rate, based on the detected electrical signal. As another example, the PPG sensor 322 may emit light toward the skin and obtain reflected light when the emitted light is reflected by the skin. The PPG sensor 322 may measure blood flow (e.g., change in the amount of blood), based on the obtained reflected light, and measure a heart rate, based on the measured blood flow. According to an embodiment, the processor 120 may measure the heart rate of the user using at least one of the ECG sensor 321 and/or the PPG sensor 322 included in the heart rate measurement sensor 320. The processor 120 may obtain heart rate information 312 of the user using at least one of the ECG sensor 321 and/or the PPG sensor 322.

According to an embodiment, the microphone 350 may be a component for obtaining an external audio signal. For example, the processor 120 may at least partially activate the microphone 350 and obtain external audio signals (e.g., user's voice signals) using the activated microphone 350.

According to an embodiment, the processor 120 may display a user interface (UI) (e.g., execution screen) for a program (e.g., health management application) related to heart rate measurement through the display 360. For example, the user interface (UI) may be implemented based on the size and/or shape of the display 360 of the electronic device 301 (e.g., a wearable device in the form of a watch) and may include information related to the user's heart rate.

According to an embodiment, the communication circuit 390 may be a component that operatively connects the electronic device 301 and another electronic device (e.g., the second electronic device 302 in FIG. 3A). For example, the processor 120 of the electronic device 301 may be operatively connected to other electronic devices through the communication circuit 390 and may transmit and receive command signals for at least partially controlling other electronic devices. According to an embodiment, the processor 120 of the electronic device 301 may transmit heart rate information measured by using the heart rate measurement sensor 320 and/or ECG-related symptoms information corresponding to the heart rate information to other electronic devices through the communication circuit 390. For example, the processor 120 may generate ECG-related data in the form of a file, based on the heart rate information, and/or ECG-related symptoms information, and may transmit the ECG-related data to other electronic devices through the communication circuit 390.

According to an embodiment, the processor 120 of the electronic device 301 may measure the heart rate of the user periodically or aperiodically using the heart rate measurement sensor 320 and determine whether or not the user has experienced symptoms related to arrhythmia, based on the measured heart rate. For example, if a symptom related to arrhythmia is identified, the processor 120 may generate at least one ECG file (e.g., data information in a form capable of being transmitted to and received from electronic devices), based on the measured heart rate and/or the identified symptom related to arrhythmia. The processor 120 may share the at least one generated ECG file with other electronic devices.

According to an embodiment, the electronic device 301 may include a heart rate measurement sensor 320, a processor 120, and a memory 130 that stores instructions. The instructions may be configured to cause, when executed by the processor 120, the electronic device 301 to obtain heart rate information by using the heart rate measurement sensor 320, identify the obtained heart rate information and ECG-related symptoms information corresponding to the heart rate information, and generate first data, based on the heart rate information and the ECG-related symptoms information.

According to an embodiment, the electronic device may further include a microphone 350, and the instructions may be configured to cause, when executed by the processor 120, the electronic device 301 to execute an audio recording function, based on the microphone 350, obtain an external audio signal, based on the executed audio recording function, identify the heart rate information measured by using the heart rate measurement sensor 320, audio information generated based on the audio signal, and the ECG-related symptoms information generated based on the heart rate information and the audio information, and generate second data, based on the heart rate information, the audio information generated based on the audio signal, and the ECG-related symptoms information.

According to an embodiment, the instructions may be configured to cause, when executed by the processor 120, the electronic device 301 to determine whether or not the audio information is at least partially included in a list of ECG symptoms stored in the memory 130 and, if the obtained audio signal is at least partially included in the list of ECG symptoms, generate the second data, based on symptoms information included in the list of ECG symptoms.

According to an embodiment, the instructions may be configured to cause, when executed by the processor 120, the electronic device 301 to generate third data, based on the heart rate information and the audio information, if the obtained audio signal is not at least partially included in the list of ECG symptoms.

According to an embodiment, the instructions may be configured to cause, when executed by the processor 120, the electronic device 301 to identify the heart rate information and the ECG-related symptoms information corresponding to the heart rate information at predetermined intervals using the heart rate measurement sensor 320, in response to (or based on) the identified ECG-related symptoms information being included in the list of ECG symptoms, display a notification message for executing an audio recording function, in response to (or based on) a user input to the notification message, execute the audio recording function, determine whether or not at least one audio signal obtained based on the audio recording function is at least partially included in the list of ECG symptoms stored in the memory 130, and if the at least one audio signal is at least partially included in the list of ECG symptoms, generate second data, based on the heart rate information measured by using the heart rate measurement sensor 320, at least one piece of audio information generated based on the at least one audio signal, and the ECG-related symptoms information generated based on the heart rate information and the at least one piece of audio information.

According to an embodiment, the instructions may be configured to cause, when executed by the processor 120, the electronic device 301 to execute the audio recording function in response to (or based on) a user input in obtaining the heart rate information and determine whether or not at least one audio signal obtained based on the audio recording function is at least partially included in a list of ECG symptoms stored in the memory 130.

According to an embodiment, the electronic device may further include a display 360, and the instructions may be configured to cause, when executed by the processor 120, the electronic device 301 to display a list of ECG symptoms through the display 360, in response to obtaining (or based on) the heart rate information, identify the ECG-related symptoms information corresponding to a user input to the list of ECG symptoms, generate the first data, based on the heart rate information measured by using the heart rate measurement sensor 320 and the ECG-related symptoms information, and display a user interface implemented based on the generated first data through the display 360.

According to an embodiment, the electronic device may further include a speaker, and the instructions may be configured to cause, when executed by the processor 120, the electronic device 301 to output at least one query sentence related to ECG-related symptoms through the speaker, in response to obtaining (or based on) the heart rate information, obtain an audio signal corresponding to the at least one query sentence using the microphone, generate at least one piece of answer information according to the at least one query sentence, based on the obtained audio signal, and generate third data, based on the heart rate information measured by using the heart rate measurement sensor 320, the at least one query sentence, and the at least one piece of answer information.

According to an embodiment, the electronic device may further include a communication circuit 390, and the instructions may be configured to cause, when executed by the processor 120, the electronic device 301 to make a call connection to a predetermined telephone number through the communication circuit 390 if the ECG-related symptoms information is included in a predetermined important symptom list (e.g., a predetermined list of major or serious health symptoms).

According to an embodiment, the instructions may be configured to cause, when executed by the processor 120, the electronic device 301 to display a notification message for the call connection through the display 360 before the call connection and, in response to (or based on) a user input to the displayed notification message, make a call connection to the predetermined telephone number.

According to an embodiment, the instructions may be configured to cause, when executed by the processor 120, the electronic device 301 to connect a call to the predetermined telephone number if a predetermined time elapses while the notification message for the call connection is displayed through the display 360.

According to an embodiment, the important symptom list may include at least one of symptoms related to shortness of breath, symptoms related to chest pressure or chest pain, or symptoms related to fainting.

According to an embodiment, the heart rate measurement sensor 320 may include an ECG sensor 321 configured to detect an electrical signal depending on a heartbeat and a PPG sensor 322 configured to measure blood flow by using light.

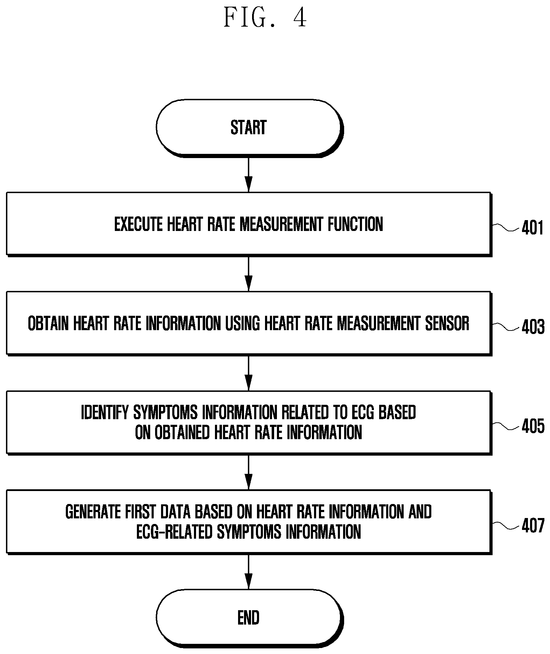

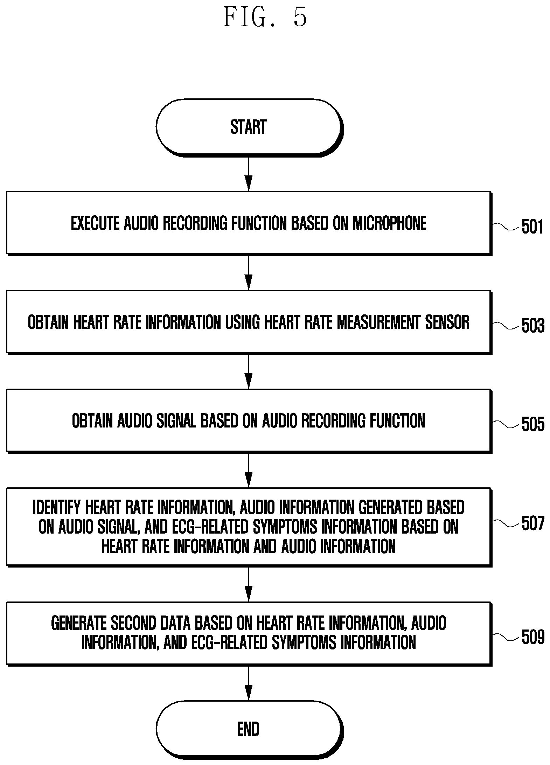

FIG. 4 is a flowchart illustrating a method of generating first data for identifying ECG-related symptoms, based on measured heart rate information, according to an embodiment of the disclosure. FIG. 5 is a flowchart illustrating a method of generating second data for identifying ECG-related symptoms, based on measured heart rate information and an external audio signal, according to an embodiment of the disclosure.

In the following embodiments, respective operations may be performed sequentially, but are not necessarily performed sequentially. For example, the sequence of the respective operations may vary, and at least two operations may be performed in parallel.

According to an embodiment, operations 401 to 407 and operations 501 to 509 may be understood to be performed by a processor (e.g., the processor 120 in FIGS. 1 and 3B) of an electronic device (e.g., the electronic device 101 in FIG. 1 or the electronic device 301 in FIG. 3B). The electronic device 301 in FIGS. 4 and 5 may be at least partially similar to the electronic device 301 in FIG. 3B, or may further include other embodiments of the electronic device 301.

According to an embodiment, the electronic device 301 may include a wearable device capable of being mounted to a part of a user's body (e.g., wrist). For example, the electronic device 301 may include a wearable watch worn on the user's wrist. The electronic device 301 may remain in at least partial contact with the user's skin when it is worn. The electronic device 301 may measure a heart rate of the user wearing the electronic device 301, based on the sensing area in physical contact with the skin. According to an embodiment, the electronic device 301 may periodically or aperiodically measure the heart rate of the user according to a predetermined time interval. For example, the electronic device 301 may execute a heart rate measurement function in response to (or based on) a heart rate measurement command signal from the user. For example, the electronic device 301 may repeatedly execute the heart rate measurement function, based on predetermined time information (e.g., time interval information).

Referring to FIG. 4, the processor 120 of the electronic device 301 may execute a heart rate measurement function in operation 401. For example, the processor 120 may execute a heart rate measurement function in response to (or based on) a user input while a program related to heart rate measurement (e.g., health management application) is running. While a user interface (e.g., execution screen) of the program related to heart rate measurement is being displayed through a display (e.g., the display 360 in FIG. 3B), the processor 120 may recognize a user input to the user interface and execute a heart rate measurement function according to the user input.

In operation 403, the processor 120 may obtain heart rate information using a heart rate measurement sensor (e.g., the heart rate measurement sensor 320 in FIG. 3B). For example, the heart rate measurement sensor 320 may be disposed in at least partial contact with the user's skin or adjacent to the skin when the electronic device 301 is worn. The heart rate measurement sensor 320 may be disposed adjacent to a portion (e.g., sensing area) of the skin. The processor 120 may detect a heartbeat corresponding to the sensing area using the heart rate measurement sensor 320 and obtain heart rate information according to the heartbeat.

In operation 405, the processor 120 may identify symptoms information related to ECG (e.g., symptoms information related to arrhythmia), based on the obtained heart rate information. For example, the processor 120 may determine whether or not the user's heartbeat is measured abnormally based on the identified heart rate information. The processor 120 may detect a situation in which the heartbeat becomes abnormally fast, slow, or irregular, and if the situation is detected, it may indicate that the heartbeat is abnormal. The processor 120 may identify symptoms information related to arrhythmia, based on heart rate information 312 and/or symptoms information 313 stored in a memory (e.g., the memory 130 in FIG. 1 and FIG. 3B).

According to an embodiment, the electronic device 301 may detect or predict arrhythmia, which is one of the diseases related to the heart rate, based on the heart rate information. For example, in the case of arrhythmia, it may be identified that the heartbeat becomes abnormally fast, slow, or irregular, and the user may feel a pounding heart, dizziness, or increased fatigue.

In operation 407, the processor 120 may generate first data, based on the heart rate information and/or symptoms information related to ECG (e.g., symptoms information related to arrhythmia). For example, the processor 120 may generate first data (e.g., information related to the user's heart rate, a file in a form capable of being shared with other electronic devices, or a first ECG file), based on the user's heart rate information measured by using the heart rate measurement sensor 320 and/or the symptoms information 313 stored in the memory 130. When the first data is executed, the processor 120 may display a user interface (UI) including the heart rate information and/or symptoms information related to arrhythmia through the display 360.

According to an embodiment, in response to (or based on) the execution of the heart rate measurement function, the electronic device 301 may generate first data including heart rate-related information (e.g., heart rate) of the user. According to an embodiment, the electronic device 301 may share the first data with other electronic devices.

Referring to FIG. 5, in operation 501, the processor 120 of the electronic device 301 may execute an audio recording function, based on a microphone (e.g., the microphone 350 in FIG. 3B). For example, the processor 120 may at least partially activate the microphone 350 while the heart rate measurement function is being executed and obtain an external audio signal using the microphone 350.

In operation 503, the processor 120 may obtain heart rate information by using the heart rate measurement sensor 320. Operation 503 may operate in the same manner as operation 403. For example, the processor 120 may detect a heartbeat corresponding to a sensing area (e.g., a portion of skin adjacent to the heart rate measurement sensor 320) using the heart rate measurement sensor 320 and obtain heart rate information according to the heartbeat.

In operation 505, the processor 120 may obtain an audio signal, based on the audio recording function. For example, the obtained audio signal may include a voice signal (e.g., uttering information or natural-language information) and/or a non-voice signal (e.g., moaning sound or voice tremor information) from the user.

In operation 507, the processor 120 may identify the heart rate information, audio information (e.g., text information) generated based on the audio signal, and ECG-related symptoms information (e.g., symptoms information related to arrhythmia) based on the heart rate information and/or the audio information. For example, the heart rate information may include a current heart rate measured by using the heart rate measurement sensor 320. If the audio signal is a voice signal, the audio information may include sentences and/or words obtained by converting the voice signal into text and recording the same. If the audio signal is a non-voice signal, the audio information may include sentences and/or words predetermined to correspond to the non-voice signal. For example, if the audio signal includes information about a trembling voice, the audio information may include sentence information such as "tachycardia or palpitations" and/or "irregular heartbeat". The ECG-related symptoms information may include at least one symptom list information (e.g., the symptoms information 313 in FIG. 3B) corresponding to arrhythmia symptoms.

In operation 509, the processor 120 may generate second data (e.g., information related to the user's heart rate, a file capable of being shared with other electronic devices, or a second ECG file), based on the heart rate information, the audio information, and/or the ECG-related symptoms information. When the second data is executed, the processor 120 may display a user interface (UI) including the heart rate information and/or the ECG-related symptoms information through the display 360.

According to an embodiment, in response to (or based on) execution of the heart rate measurement function, the electronic device 301 may generate second data including the user's heart rate-related information (e.g., heart rate) and/or the audio information (e.g., text information). According to an embodiment, the electronic device 301 may share the second data with other electronic devices. For example, when executing the second data in another electronic device, the corresponding electronic device may display a user interface implemented based on at least one of the heart rate information, the audio information, and/or the ECG-related symptoms information included in the second data.

According to an embodiment, the electronic device 301 may repeatedly execute the heart rate measurement function according to a configured cycle (e.g., about 10 minutes). For example, the processor 120 of the electronic device 301 may execute an audio recording function through the microphone 350 while executing the heart rate measurement function about every 10 minutes. According to an embodiment, the electronic device 301 may obtain about six pieces of heart rate information and about six audio signals for about 1 hour. The electronic device 301 may identify a situation in which the heart rate information and/or the audio signal changes, based on the predetermined cycle, and generate data (e.g., an ECG file) including a plurality of pieces of heart rate information and/or a plurality of audio signals.

According to an embodiment, the electronic device 301 may execute the heart rate measurement function according to a predetermined cycle (e.g., about 10 minutes) to identify the heart rate information and the ECG-related symptoms information corresponding to the heart rate information. If the identified ECG-related symptoms information is included in a list of ECG symptoms (e.g., if an abnormal symptom related to ECG is detected), the electronic device 301 may automatically generate and display a notification message for executing the audio recording function. The electronic device 301 may execute the audio recording function in response to (or based on) a user input for the notification message being displayed and obtain a user's voice signal (e.g., audio signal) by the audio recording function. The electronic device 101 may generate data (e.g., an ECG file) including the heart rate information and the audio signal, based on the ECG-related symptoms information generated based on the obtained voice signal. According to an embodiment, the electronic device 301 may periodically check the heart rate information by the heart rate measurement function and, if the heart rate information belongs to a list of ECG symptoms corresponding to abnormal symptoms, output a notification message for executing the audio recording function. in response to (or based on) a situation in which the heart rate information corresponds to an abnormal symptom, the electronic device 301 may automatically provide the user with a notification message for the audio recording function.

According to an embodiment, the electronic device 301 may execute an audio recording function, based on a user input, when obtaining the heart rate information. For example, if the user feels discomfort in relation to the heart rate, the user may perform heart rate measurement, based on the audio recording function. The electronic device 301 may execute the audio recording function in response to (or based on) a user input that generates an execution command of the audio recording function, and may obtain a user's voice signal (e.g., audio signal) by the audio recording function. The electronic device 101 may generate data (e.g., an ECG file) including the heart rate information and the audio signal, based on the ECG-related symptoms information generated based on the obtained voice signal.

According to an embodiment, the electronic device 301 may share the ECG file with another external electronic device. For example, the electronic device 301 may generate data (e.g., an ECG file) including heart rate-related information corresponding to the user who is a patient and share the data (e.g., an ECG file) with another electronic device used by a doctor during a consultation with the doctor.

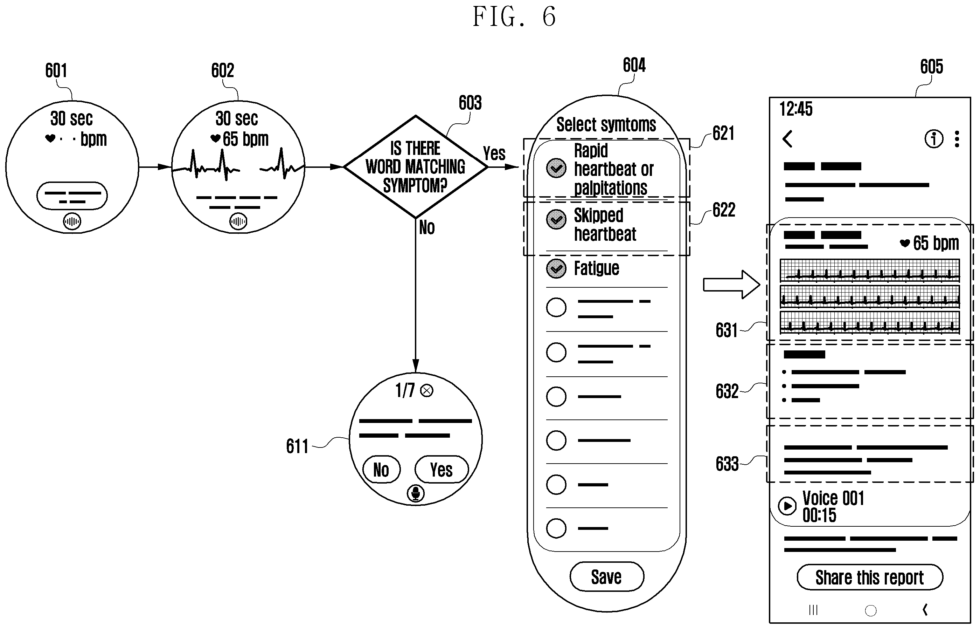

FIG. 6 is an example diagram illustrating operations in respective steps in the process of generating an ECG file according to an embodiment of the disclosure.

The electronic device 301 (e.g., the electronic device 101 in FIG. 1 or the electronic device 301 in FIG. 3B) in FIG. 6 may be at least partially similar to the first electronic device 301 in FIG. 3A, or may further include other embodiments of the first electronic device. A second electronic device 302 (e.g., a smartphone or a portable terminal) in FIG. 6 may be at least partially similar to the electronic device 102 or 104 in FIG. 1, or may further include other embodiments of the electronic device 102 or 104. The operations illustrated in FIG. 6 may be performed in at least one of the first electronic device 301 and/or the second electronic device 302. According to an embodiment, some (e.g., an operation of measuring heart rate information) of the operations illustrated in FIG. 6 may be performed in the first electronic device 301, and others (e.g., an operation of generating data related to a heart rate, based on the measured heart rate information) of the operations illustrated in FIG. 6 may be performed in the second electronic device 302. According to an embodiment, the operations illustrated in FIG. 6 may be performed independently in each of the first electronic device 301 and the second electronic device 302.

According to an embodiment, the electronic device (e.g., the first electronic device 301 in FIG. 3a, or the electronic device 301 in FIG. 3b) may include a wearable device capable of being mounted to a part (e.g., wrist) of the user's body. For example, the electronic device 301 may include a wearable watch worn on the user's wrist. The electronic device 301 may remain in at least partial contact with the user's skin when it is worn. The electronic device 301 may measure a heart rate of the user wearing the electronic device 301, based on the sensing area in physical contact with the skin. According to an embodiment, the wearable watch (the smart watch) illustrated in FIGS. 2A and 2B are not the only example of the electronic device 301 described in the disclosure. Other form factors that can be worn by or attached to the user, such as the smart ring, can be other examples of the electronic device 301. According to an embodiment, the electronic device 301 may periodically or aperiodically measure the heart rate of the user according to a predetermined time interval. For example, the electronic device 301 may execute a heart rate measurement function in response to (or based on) a heart rate measurement command signal from the user. For example, the electronic device 301 may repeatedly execute the heart rate measurement function, based on predetermined time information (e.g., time interval information).

Referring to FIG. 6, a first screen 601 may include a user interface displayed on a display (e.g., the display 360 in FIG. 3B) of the electronic device 301. For example, the processor (e.g., the processor 120 in FIG. 3B) of the electronic device 301 may display a user interface for heart rate measurement in response to (or based on) execution of a program (e.g., health management application) related to heart rate measurement.

A second screen 602 may include a user interface changed when the heart rate measurement function is executed in the electronic device 301. When executing the heart rate measurement function, the processor 120 may measure the user's heart rate using a heart rate measurement sensor (e.g., the heart rate measurement sensor 320 in FIG. 3B) and display a user interface indicating that the heart rate is being measured. According to an embodiment, the electronic device 301 may activate a microphone (e.g., the microphone 350 in FIG. 3B) in response to (or based on) the execution of the heart rate measurement function and obtain an audio signal (e.g., a user's voice signal) from the surroundings using the activated microphone. The processor 120 may generate audio information (e.g., data in the form of text) based on the obtained audio signal. For example, the audio signal may include natural language spoken by the user, and the audio information may include information obtained by converting the audio signal into text.

In operation 603, the processor 120 may determine whether or not the generated audio information includes a word matching symptoms related to ECG (e.g., symptoms information related to arrhythmia). For example, if the audio information is "the heartbeat is irregular", the words "heart, heartbeat, and irregular" may belong to the symptoms related to arrhythmia. According to an embodiment, if the audio information includes a word corresponding to the symptoms related to arrhythmia, the processor 120 may select a symptom corresponding to the word from the symptom list on a fourth screen 604. The processor 120 may select at least one symptom including the word corresponding to the audio information from the symptom list including a plurality of symptoms.

If the audio information does not include a word corresponding to symptoms related to arrhythmia in operation 603, the processor 120 may provide the user with a query sentence related to arrhythmia on a third screen 611. For example, the processor 120 may output the query sentence related to arrhythmia using a speaker. If the user selects an answer to the query sentence, the processor 120 may select at least one symptom corresponding to the selected answer.

The fourth screen 604 may include a user interface in which a first symptom 621 (e.g., "rapid heartbeat or palpitations", tachycardia, and/or palpitations), a second symptom 622 (e.g., "skipped heartbeat", skipped heartbeat, or irregular heartbeat), and/or a third symptom (e.g., "fatigue" or feeling tired) is selected from the symptom list including a plurality of pieces of symptoms information in the electronic device 301. The fourth screen 604 may display some of a plurality of symptom lists by a drag input (e.g., a scroll input). The processor 120 may perform control such that some of the plurality of symptom lists are displayed through the display 360.

According to an embodiment, the electronic device 301 may generate an ECG file, based on heart rate information measured on the second screen 602, audio information generated based on an audio signal obtained on the second screen 602, and/or symptoms information selected on the fourth screen 604. The electronic device 301 may share the ECG file with another electronic device connected thereto.

According to an embodiment, when executing the ECG file, the processor 120 may display a user interface 605 including a heart rate graph 631 corresponding to the heart rate information, audio information 632 obtained by converting the audio signal into text, and/or symptoms information 633 selected based on the audio information 632.

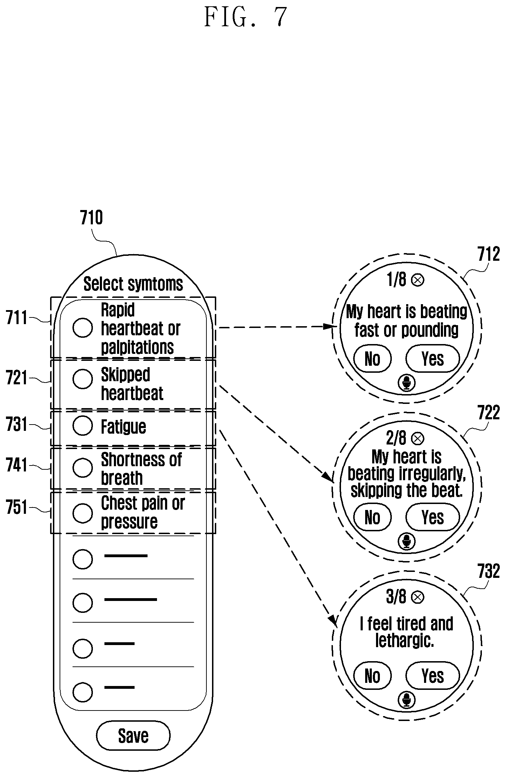

FIG. 7 is an example diagram illustrating an operation of outputting predetermined questions in relation to ECG symptoms according to an embodiment of the disclosure.

The electronic device 301 (e.g., the electronic device 101 in FIG. 1 or the electronic device 301 in FIG. 3B) in FIG. 7 may be at least partially similar to the first electronic device 301 in FIG. 3A, or may further include other embodiments of the first electronic device.

According to an embodiment, the electronic device 301 may include a wearable device capable of being mounted to a part (e.g., wrist) of the user's body. For example, the electronic device 301 may include a wearable watch worn on the user's wrist.

Referring to FIG. 7, a symptom list 710 including a plurality of pieces of symptoms information 711, 721, 731, 741, and 751 is illustrated. The electronic device 301 may execute an audio recording function to generate audio information, based on an obtained audio signal, and may determine whether or not words and/or sentences included in the generated audio information are at least partially included in the symptoms information 711, 721, 731, 741, and 751. If words and/or sentences included in the audio information are not included in the symptoms information 711, 721, 731, 741, and 751, the electronic device 301 may generate a query sentence corresponding to each piece of symptoms information.

For example, the electronic device 301 may be in the state in which a first query sentence 712 (e.g., "My heart is beating fast or pounding.") for selecting a first symptom 711 (e.g., "Rapid heartbeat or palpitations") is predetermined, and may output the first query sentence 712 using a speaker and display the first query sentence 712 through a display 360. The electronic device 301 may obtain an answer (e.g., audio signal) to the first query sentence 712 using a microphone 350 or obtain an answer by a user's touch input. If it is identified that the symptom felt by the user is the first symptom 711, based on the answer to the first query sentence 712, the processor 120 may select the first symptom 711.

For example, the electronic device 301 may be in the state in which a second query sentence 722 (e.g., "My heart is beating irregularly or skipping the beat.") for selecting a second symptom 721 (e.g., "Skipped heartbeat") is predetermined, and may output a second query sentence 722 using the speaker.

For example, the electronic device 301 may be in the state in which a third query sentence 732 (e.g., "I feel tired and lethargic.") for selecting a third symptom 731 (e.g., "Fatigue") is predetermined, and may output a third query sentence 732 using the speaker.

According to an embodiment, the electronic device 301 may provide the user with a query sentence corresponding to each piece of symptoms information, based on a symptom list including a plurality of pieces of symptoms information related to ECG, and select symptoms information related to arrhythmia, based on a user's answer to the query sentence. For example, the electronic device 301 may identify whether or not the symptom of the user corresponds to arrhythmia-related symptoms.

According to an embodiment, the electronic device 301 may generate an ECG file representing symptoms information related to arrhythmia, based on a plurality of answers from the user corresponding to a plurality of query sentences. The electronic device 301 may share the ECG file with another electronic device.

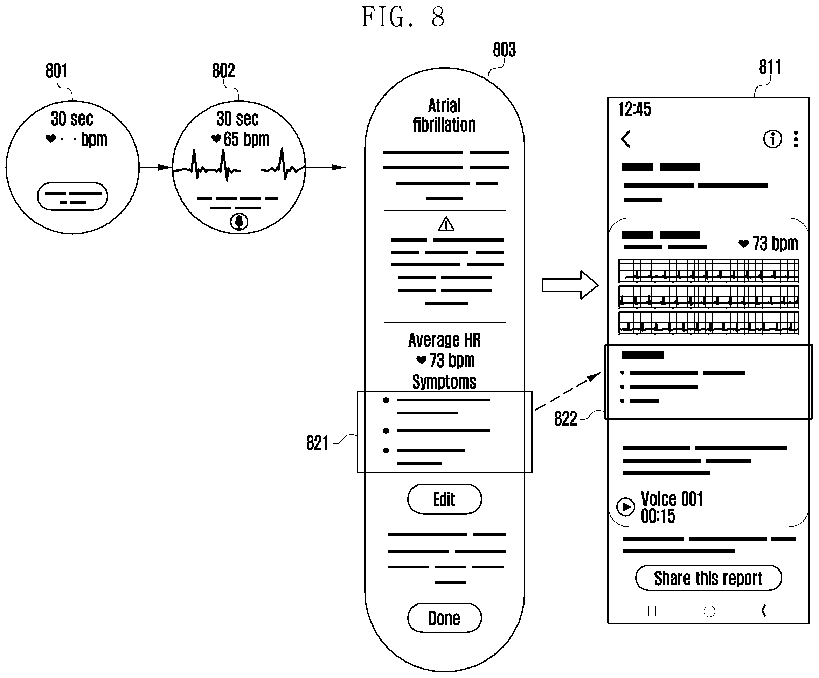

FIG. 8 is an example diagram illustrating an operation of obtaining an audio signal of a user for a question related to ECG symptoms and recording ECG symptoms, based on the obtained user's audio signal, according to an embodiment of the disclosure.

The electronic device 301 (e.g., the electronic device 101 in FIG. 1 or the electronic device 301 in FIG. 3B) in FIG. 8 may be at least partially similar to the first electronic device 301 in FIG. 3A, or may further include other embodiments of the first electronic device. A second electronic device 302 (e.g., a smartphone or a portable terminal) in FIG. 8 may be at least partially similar to the electronic device 102 or 104 in FIG. 1, or may further include other embodiments of the electronic device 102 or 104. The operations illustrated in FIG. 8 may be performed in at least one of the first electronic device 301 and/or the second electronic device 302. According to an embodiment, some (e.g., an operation of measuring heart rate information) of the operations illustrated in FIG. 8 may be performed in the first electronic device 301, and others (e.g., an operation of generating data related to a heart rate, based on the measured heart rate information) of the operations illustrated in FIG. 8 may be performed in the second electronic device 302. According to an embodiment, the operations illustrated in FIG. 8 may be performed independently in each of the first electronic device 301 and the second electronic device 302.

According to an embodiment, the electronic device (e.g., the first electronic device 301 in FIG. 3a, or the electronic device 301 in FIG. 3b) may include a wearable device capable of being mounted to a part (e.g., wrist) of the user's body. For example, the electronic device 301 may include a wearable watch worn on the user's wrist. According to an embodiment, the electronic device 301 may periodically or aperiodically measure the heart rate of the user according to a predetermined time interval. According to an embodiment, the wearable watch (the smart watch) illustrated in FIGS. 2A and 2B are not the only example of the electronic device 301 described in the disclosure. Other form factors that can be worn by or attached to the user, such as the smart ring, can be other examples of the electronic device 301.

Referring to FIG. 8, the first screen 801 may include a user interface displayed through a display (e.g., display 360 in FIG. 3B) of the electronic device 301. For example, the processor (e.g., processor 120 in FIG. 3B) of the electronic device 301 may display a user interface for heart rate measurement in response to (or based on) execution of a program related to heart rate measurement (e.g., health management application).

The second screen 802 may include a user interface changed when a heart rate measurement function is executed in the electronic device 301. The processor 120 may measure a user's heart rate using a heart rate measurement sensor (e.g., the heart rate measurement sensor 320 in FIG. 3B) and display a user interface indicating that the heart rate is being measured. According to an embodiment, the electronic device 301 may activate a microphone (e.g., the microphone 350 in FIG. 3B) in response to (or based on) the execution of the heart rate measurement function and obtain an audio signal from the surroundings using the activated microphone. For example, the audio signal may include a non-voice signal (e.g., voice tremor information, moaning sound, breathing sound, change in tone, and sound related to a fall) of the user.

The electronic device 301 may recognize the non-voice signal by the user, and may identify at least one piece of symptoms information predetermined corresponding to the non-voice signal. For example, the symptoms information may be included in a symptom list including a plurality of symptoms related to arrhythmia. For example, if "voice tremor information" is identified based on the non-voice signal, the processor 120 may identify that the user is in the state of tachycardia (e.g., a relatively rapid heart rate) and/or has an irregular heartbeat. In the case of the tachycardia state and/or irregular heartbeat state, the processor 120 may select at least one symptom (e.g., the first symptom 711 in FIG. 7) from among a plurality of symptoms. For example, if "panting or wheezing" is identified based on the non-voice signal, the processor 120 may determine that the user is in the state of tachycardia (e.g., a relatively fast heart rate) and/or is experiencing difficulty breathing. In the case of the tachycardia state and/or difficulty breathing, the processor 120 may select the fourth symptom 741 in FIG. 7 from among the plurality of symptoms. For example, if "moaning sound" is identified based on the non-voice signal, the processor 120 may determine that the user is experiencing chest pain or feeling pressure. If the user feels chest pain and/or pressure, the processor 120 may select the fifth symptom 751 in FIG. 7 from among the plurality of symptoms.

According to an embodiment, the processor 120 may identify symptoms information 821 (e.g., arrhythmia-related symptoms information) corresponding to the non-voice signal obtained through the microphone 350 from the symptom list including a plurality of symptoms related to arrhythmia, and select the identified symptoms information 821.

The third screen 803 may include a user interface in which symptoms information 821 related to arrhythmia is selected in the electronic device 301 and displayed through the display 360. According to an embodiment, the electronic device 301 may generate ECG-related data (e.g., an ECG file), based on the user's heart rate information measured by using the heart rate measurement sensor 320 and/or arrhythmia-related symptoms information 821 included in the third screen 803. The electronic device 301 may share the ECG-related data with another electronic device connected thereto.

According to an embodiment, when executing the ECG-related data, the processor 120 may display a user interface 811 including a heart rate graph corresponding to the heart rate information and arrhythmia-related symptoms information 822 selected based on the audio signal (e.g., non-voice signal).

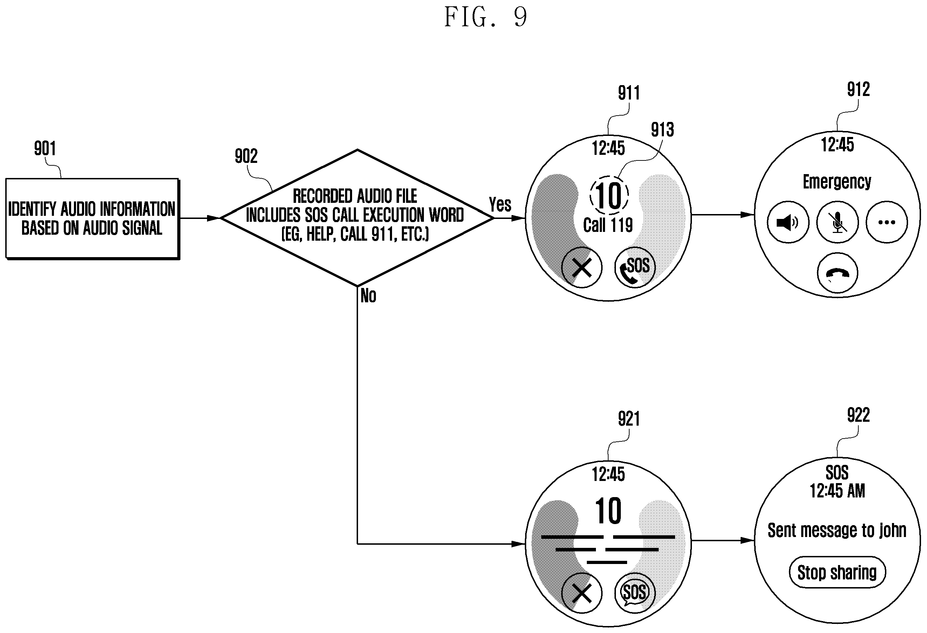

FIG. 9 is an example diagram illustrating an operation of identifying a predetermined telephone number according to an audio signal of a user and requesting a call to the predetermined telephone number according to an embodiment of the disclosure.

The electronic device 301 (e.g., the electronic device 101 in FIG. 1 or the electronic device 301 in FIG. 3B) in FIG. 9 may be at least partially similar to the first electronic device 301 in FIG. 3A, or may further include other embodiments of the first electronic device.

According to an embodiment, the electronic device 301 may include a wearable device capable of being mounted to a part (e.g., wrist) of the user's body. For example, the electronic device 301 may include a wearable watch worn on the user's wrist.

Referring to FIG. 9, in operation 901, a processor (e.g., the processor 120 in FIG. 3B) of the electronic device 301 may identify audio information, based on an audio signal. For example, the electronic device 301 may activate a microphone (e.g., the microphone 350 in FIG. 3B) in response to (or based on) the execution of the heart rate measurement function and obtain an audio signal (e.g., a user's voice signal) from the surroundings using the activated microphone. The processor 120 may identify audio information (e.g., data in the form of text), based on the obtained audio signal.

In operation 902, the processor 120 may determine whether or not the identified audio information includes words related to an "emergency call request" (e.g., SOS call, call 911, help, emergency, or emergency call). For example, the words related to an "emergency call request" may be predetermined and stored in a memory (e.g., the memory 130 in FIG. 3B). The processor 120 may determine whether or not words and/or sentences in the form of text included in the audio information at least partially match words related to an "emergency call request."

If "emergency call request" is identified in operation 902, the processor 120 may display a first screen 911 (e.g., a notification screen before connecting an emergency call) through a display (e.g., the display 360 in FIG. 3B). For example, the first screen 911 may display count information 913 and/or an icon for determining an emergency call. Although the count information 913 is predetermined as "10 (seconds)" in the first screen 911, it is not limited thereto. If the count information 913 is reduced from "10 (seconds)" to "0 (seconds)", the processor 120 may connect an emergency call. If a user input is received to a call icon before the count information 913 drops to "0 (seconds)", the processor 120 may connect an emergency call. When an emergency call is connected, the processor 120 may display a second screen 912 through the display 360.

If the "emergency call request" is not identified in operation 902, the processor 120 may display a third screen 921 (e.g., a notification screen before sending an emergency message) through the display (e.g., the display 360 in FIG. 3B). For example, the third screen 921 may display count information and/or an icon for sending an emergency message. Although count information is predetermined as "10 (seconds)" in the third screen 921, it is not limited thereto. When the count information drops from "10 (seconds)" to "0 (seconds)", the processor 120 may send an emergency message. If a user input is received to a message sending icon before the count information drops to "0 (seconds)", the processor 120 may send an emergency message. When sending an emergency message, the processor 120 may display a fourth screen 922 through the display 360.

According to an embodiment, the electronic device 301 may determine whether or not arrhythmia-related symptoms have occurred based on the identified audio information. In particular, the processor 120 may distinguish at least one symptom among the symptoms related to arrhythmia as a serious symptom (e.g., "shortness of breath", "chest pain or pressure", or "fainting"). According to an embodiment, if the occurrence of a serious symptom is identified based on the identified audio information, the processor 120 may perform an "emergency call". The processor 120 may identify a phone number (e.g., 112 or 119) related to the "emergency call" and attempt to connect a call to the identified phone number. According to an embodiment, when an "emergency call request" is made, the electronic device 301 may attempt to connect a call to a specific phone number (e.g., a guardian's phone number) predetermined by the user, instead of the phone number related to the "emergency call", or may send an emergency message thereto.

A method for generating ECG-related data according to an embodiment may include obtaining heart rate information using a heart rate measurement sensor 320, identifying the obtained heart rate information and ECG-related symptoms information corresponding to the heart rate information, and generating first data, based on the heart rate information and the ECG-related symptoms information.

The method according to an embodiment may include executing an audio recording function, based on a microphone 350, obtaining an external audio signal, based on the executed audio recording function, identifying the heart rate information measured by using the heart rate measurement sensor 320, audio information generated based on the audio signal, and the ECG-related symptoms information generated based on the heart rate information and the audio information, and generating second data, based on the heart rate information, the audio information generated based on the audio signal, and the ECG-related symptoms information.

The method according to an embodiment may include determining whether or not the audio information is at least partially included in a list of ECG symptoms, generating the second data, based on symptoms information included in the list of ECG symptoms if the obtained audio signal is at least partially included in the list of ECG symptoms, and generating third data, based on the heart rate information and the audio information if the obtained audio signal is not at least partially included in the list of ECG symptoms.

The method according to an embodiment may include displaying a list of ECG symptoms through a display 360 in response to obtaining (or based on) the heart rate information, identifying the ECG-related symptoms information corresponding to a user input to the list of ECG symptoms, generating the first data, based on the heart rate information measured by using the heart rate measurement sensor 320 and the ECG-related symptoms information, and displaying a user interface implemented based on the generated first data through the display 360.

The method according to an embodiment may include outputting at least one query sentence related to ECG-related symptoms through a speaker in response to obtaining (or based on) the heart rate information, obtaining an audio signal corresponding to the at least one query sentence using a microphone 350, generating at least one piece of answer information according to the at least one query sentence, based on the obtained audio signal, and generating third data, based on the heart rate information measured by using the heart rate measurement sensor 320, the at least one query sentence, and the at least one piece of answer information.

The method according to an embodiment may include making a call to a predetermined telephone number through a communication circuit 390 if the ECG-related symptoms information is included in a predetermined important symptom list.

The method according to an embodiment may include displaying a notification message for the call connection through the display 360 before the call connection and, in response to (or based on) a user input to the displayed notification message, making a call to the predetermined telephone number.

According to an embodiment, a non-transitory computer-readable storage medium (or computer program product) storing one or more programs for performing a method for generating an ECG-related data may be described. According to an embodiment, the one or more programs may include instructions configured to perform, when executed by a processor of an electronic device, obtaining heart rate information using a heart rate measurement sensor, identifying the obtained heart rate information and ECG-related symptoms information corresponding to the heart rate information; and generating first data, based on the heart rate information and the ECG-related symptoms information.

The electronic device according to various embodiments may be one of various types of electronic devices. The electronic devices may include, for example, a portable communication device (e.g., a smartphone), a computer device, a portable multimedia device, a portable medical device, a camera, a wearable device, a home appliance, or the like. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. It is intended that features described with respect to separate embodiments, or features recited in separate claims, may be combined unless such a combination is explicitly specified as being excluded or such features are incompatible. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), the element may be coupled with the other element directly (e.g., wiredly), wirelessly, or via a third element.

As used in connection with various embodiments of the disclosure, the term "module" may include a unit implemented in hardware, software, or firmware, or any combination thereof, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the "non-transitory" storage medium is a tangible device, and may not include a signal (e.g., an electromagnetic wave), but this term does not differentiate between where data is semipermanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStoreTM), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. An electronic device comprising:
a heart rate measurement sensor;
at least one processor; and
at least one memory including computer program code, where the at least one memory and the computer program code are configured, with the at least one processor, to cause the electronic device to at least:
obtain heart rate information by using the heart rate measurement sensor,
identify the obtained heart rate information and electrocardiogram (ECG)-related symptoms information corresponding to the heart rate information, and
generate first data, based on the heart rate information and the ECG-related symptoms information.

2. The electronic device of claim 1, further comprising a microphone,
wherein the at least one memory and the computer program code are further configured, with the at least one processor, to cause the electronic device to:
execute an audio recording function, based on the microphone;
obtain an audio signal, based on the executed audio recording function;
identify the heart rate information measured by using the heart rate measurement sensor, audio information generated based on the audio signal, and the ECG-related symptoms information generated based on the heart rate information and the audio information; and
generate second data, based on the heart rate information, the audio information generated based on the audio signal, and the ECG-related symptoms information.

3. The electronic device of claim 2, wherein the at least one memory and the computer program code are further configured, with the at least one processor, to cause the electronic device to:
determine whether the audio information is at least partially included in a list of ECG symptoms stored in the at least one memory, and
when the obtained audio signal is at least partially included in the list of ECG symptoms, generate the second data, based on symptoms information included in the list of ECG symptoms.

4. The electronic device of claim 3, wherein the at least one memory and the computer program code are further configured, with the at least one processor, to cause the electronic device to generate third data, based on the heart rate information and the audio information, when the obtained audio signal is not at least partially included in the list of ECG symptoms.

5. The electronic device of claim 3, wherein the at least one memory and the computer program code are further configured, with the at least one processor, to cause the electronic device to:
identify the heart rate information and the ECG-related symptoms information corresponding to the heart rate information at predetermined intervals using the heart rate measurement sensor;
based on the identified ECG-related symptoms information being included in the list of ECG symptoms, display a notification message for executing the audio recording function;
based on a user input to the notification message, execute the audio recording function;
when the audio recording function is at least partially included in the list of ECG symptoms stored in the at least one memory, determine whether at least one audio signal is obtained; and
when the at least one audio signal is at least partially included in the list of ECG symptoms, generate the second data, based on:
the heart rate information measured by using the heart rate measurement sensor,
at least one piece of audio information generated based on the at least one audio signal, and
the ECG-related symptoms information generated based on the heart rate information and the at least one piece of audio information.

6. The electronic device of claim 2, wherein the at least one memory and the computer program code are further configured, with the at least one processor, to cause the electronic device to:
execute the audio recording function based on a user input in obtaining the heart rate information; and
determine whether or not at least one audio signal obtained based on the audio recording function is at least partially included in a list of ECG symptoms stored in the at least one memory.

7. The electronic device of claim 1, further comprising a display,
wherein the at least one memory and the computer program code are further configured, with the at least one processor, to cause the electronic device to:
display a list of ECG symptoms through the display based on the heart rate information;
identify the ECG-related symptoms information corresponding to a user input to the list of ECG symptoms;
generate the first data, based on the heart rate information measured by using the heart rate measurement sensor and the ECG-related symptoms information; and
display a user interface based on the generated first data through the display.

8. The electronic device of claim 2, further comprising a speaker,
wherein the at least one memory and the computer program code are further configured, with the at least one processor, to cause the electronic device to:
output at least one query sentence related to ECG-related symptoms through the speaker based on the heart rate information;
obtain an audio signal corresponding to the at least one query sentence using the microphone;
generate at least one piece of answer information based on the at least one query sentence and the obtained audio signal; and
generate third data, based on the heart rate information measured by using the heart rate measurement sensor, the at least one query sentence, and the at least one piece of answer information.

9. The electronic device of claim 1, further comprising a communication circuit,
wherein the at least one memory and the computer program code are further configured, with the at least one processor, to cause the electronic device to make a call connection to a predetermined telephone number through the communication circuit when the ECG-related symptoms information is included in a predetermined important symptom list.

10. The electronic device of claim 9, wherein the at least one memory and the computer program code are further configured, with the at least one processor, to cause the electronic device to:
display a notification message for the call connection through the display before the call connection; and
based on a user input to the displayed notification message, make the call connection to the predetermined telephone number.

11. The electronic device of claim 10, wherein the at least one memory and the computer program code are further configured, with the at least one processor, to cause the electronic device to make the call connection to the predetermined telephone number when a predetermined time elapses and the notification message for the call connection is displayed through the display.

12. The electronic device of claim 9, wherein the predetermined important symptom list comprises at least one of symptoms related to shortness of breath, symptoms related to chest pressure or chest pain, or symptoms related to fainting.

13. The electronic device of claim 1, wherein the heart rate measurement sensor comprises an ECG sensor configured to detect an electrical signal depending on a heartbeat and a photoplethysmography (PPG) sensor configured to measure blood flow by using light.

14. A method for generating data related to electrocardiogram, the method comprising:
obtaining heart rate information using a heart rate measurement sensor;
identifying the obtained heart rate information and ECG-related symptoms information corresponding to the heart rate information; and
generating first data, based on the heart rate information and the ECG-related symptoms information.

15. A non-transitory computer-readable storage medium storing one or more programs for performing a method for generating an ECG-related file, wherein the one or more programs comprise instructions configured to, when executed by at least one processor of an electronic device, perform:
obtaining heart rate information using a heart rate measurement sensor;
identifying the obtained heart rate information and ECG-related symptoms information corresponding to the heart rate information; and
generating first data, based on the heart rate information and the ECG-related symptoms information.
